# EUROPEAN PATENT APPLICATION

(11) **EP 3 858 373 A1**
(43) Date of publication of application: **04.08.2021**
(21) Application number: 20154980.5
(22) Date of filing: 31.01.2020
(51) Int. Cl.: A61K 38/22, A61K 38/26, A61K 45/06, A61P 3/04

(54) **COMPOSITIONS COMPRISING AT LEAST AN AMYLIN RECEPTOR AGONIST AND A GLP-1 RECEPTOR AGONIST**

(71) Applicant: Adocia, 69003 Lyon (FR)
(72) Inventor: CHAN, You-Ping, 69360 TERNAY (FR); MEIFFREN, Grégory., 69330 MEYZIEU (FR)
(74) Representative: Tripoz, Inès

(57) **Abstract**

The invention relates to therapies for treating obesity, overweight and/or diabetes, in particular type 2 diabetes.

The invention relates to a composition comprising at least one amylin receptor agonist, such as pramlintide, and at least one GLP-1 receptor agonist, such as exenatide or lixisenatide.

The invention also relates to a composition for use in the method for the treatment of obesity, overweight and/or diabetes, in particular type 2 diabetes, characterized in that it reduces the slowing effect of gastric emptying of amylin RA as compared to the same composition without the corresponding GLP-1 RA.

## Description

The invention relates to therapies for treating obesity, overweight and/or diabetes, in particular type 2 diabetes.

In recent times, obesity has spread rapidly in the world with the US and China being the most affected population. Today, 650 million of adults (13% of adults) and about 124 million of children older than 5 are obese in the world. In the US, 42.8% of adults and 18.5% of children are obese.

It has major health implications that can cause premature death due the increased risk of developing several diseases including type II diabetes, cerebrovascular disease, sleep apnea, cancers.

A weight loss of 10 to 15% is thought to significantly reduce the incidence of severe complications.

To treat this disease, therapies have focused on reducing food intake in patients.

Diet and physical activity are the foundations for treating obesity, but the use of medications is becoming a prevalent strategy for a long-term success over this disease.

Amylin receptor agonists are useful to reduce food intake and treating obesity.

Amylin receptor agonists and more particularly Pramlintide, a human amylin analog, have been identified to diminish the food intake but it slows down gastric emptying.

GLP-1 receptor agonist (GLP-1 RA), are also known as incretin mimetics are insulin secretagogues. Among GLP-1 RA can be cited exenatide and lixisenatide, which are also known for diminishing the food intake and for slowing down the gastric emptying.

Pramlintide and GLP-1 RA, such as exenatide and lixisenatide, have several side effects, for example nausea, which may cause a problem of tolerability.

Surprisingly, the applicant has demonstrated that a composition containing pramlintide in combination with exenatide allows reducing the slow-down of gastric emptying in comparison to an identical composition without exenatide. These compositions may thus exhibit a better tolerability profile.

The invention relates to a composition comprising at least one amylin receptor agonist, such as pramlintide, and at least one GLP-1 receptor agonist, such as exenatide or lixisenatide.

The invention also concerns a composition comprising pharmaceutical active ingredients (API) chosen in the group comprising pramlintide and a GLP-1 receptor agonist (GLP-1 RA) as only pharmaceutical ingredients.

Pramlintide is an amylin analog which has been developed by the company Amylin to compensate the lack of physical stability of human amylin. This product, marketed under the name of Symlin®, was approved in 2005 by the FDA for the treatment of type 1 and type 2 diabetes, as a complement to insulin therapy.

Pramlintide is a peptide having the sequence:
KCNTATCATQRLANFLVHSSNNFGPILPPTNVGSNTY (SEQ ID n°1)

Similarly to amylin, human GLP-1 cannot be used as a therapeutic treatment due to its extremely short half-life. Various GLP-1 derivatives, GLP-1 receptor agonists, referred to as GLP-1 RA, or GLP-1 analogs reproduce the effects of GLP-1, while having a longer half-life. One such FDA drug is Byetta® (exenatide) which is a synthetic product of exendin-4.

GLP-1 is also described to play a similar role to that of amylin as a meal-induced inhibitory signal.

In an embodiment, the GLP-1, GLP-1 analogs, or GLP-1 RA are "short-acting" or "prandial." "Short-acting" or "prandial" is understood to mean GLP-1, GLP-1 analogs, or GLP-1 RA of which the apparent half-life of elimination after subcutaneous injection in humans is less than 8 hours, in particular less than 5 hours, preferably less than 4 hours or else less than 3 hours, such as, for example, exenatide or lixisenatide.

In an embodiment, the GLP-1, the GLP-1 analogs, or the GLP-1 RA are selected from the group consisting of exenatide (Byetta®, ASTRA-ZENECA), lixisenatide (Lyxumia®, SANOFI), the analogs or derivatives thereof and pharmaceutically acceptable salts thereof.

Exenatide is a peptide having the sequence:
HGEGTFTSDL SKQMEEEAVR LFIEWLKNGG PSSGAPPPS (SEQ ID n°2)

Lixisenatide is a peptide having the sequence:
HGEGTFTSDL SKQMEEEAVR LFIEWLKNGG PSSGAPPSKK KKKK-[NH2] (SEQ ID n°3)

Exenatide and lixisenatide, are described in the applications US2004/0023871 and WO0104156, entirely included herein by reference.

In an embodiment, the GLP-1, the GLP-1 analog, or GLP-1 RA is exenatide or Byetta®, analogs or derivatives thereof and pharmaceutically acceptable salts thereof.

In an embodiment, the GLP-1, the GLP-1 analog, or GLP-1 RA is lixisenatide or Lyxumia®, analogs or derivatives thereof and pharmaceutically acceptable salts thereof.

In an embodiment the invention concerns, a composition comprising pharmaceutical active ingredients (API) chosen in the group consisting of pramlintide and a GLP-1 receptor agonist (GLP-1 RA).

In an embodiment the invention relates to a composition, characterized in that it comprises only amylin RA and GLP-RA as API.

In an embodiment the invention relates to a composition, characterized in that it comprises only pramlintide and exenatide as API.

In an embodiment the invention relates to a composition, characterized in that it comprises only pramlintide and lixisenatide as API.

In an embodiment the invention concerns a composition characterized that the GLP-1 receptor agonist (GLP-1 RA) is chosen amongst lixisenatide and exenatide.

In one embodiment the GLP-1 receptor agonist (GLP-1 RA) is lisixenatide.

In one embodiment the GLP-1 receptor agonist (GLP-1 RA) is exenatide.

The invention also relates to a composition comprising pharmaceutical active ingredients (API) chosen in the group consisting of pramlintide and exenatide.

The invention also relates to a composition comprising pharmaceutical active ingredients (API) chosen in the group consisting of pramlintide and lixisenatide.

The invention also relates to stable pharmaceutical formulation including such composition.

In an embodiment the invention relates to a composition, characterized in that it is in the form of an aqueous solution.

In an embodiment the invention relates to a composition, characterized in that it is in the form of an injectable aqueous solution.

In an embodiment the invention relates to a composition, characterized in that it is in the form of an injectable aqueous formulation.

Such formulation is typically a solution or a suspension.

In a further embodiment of the invention the pharmaceutical formulation is an aqueous solution.

The term "aqueous formulation" is defined as a formulation comprising at least 50 %w/w water.

Likewise, the term "aqueous solution" is defined as a solution comprising at least 50 %w/w water, and the term "aqueous suspension" is defined as a suspension comprising at least 50 %w/w water.

The compositions in the form of an injectable aqueous solution according to the invention are clear solutions. "Clear solution" is understood to mean compositions which satisfy the criteria described in the American and European pharmacopoeias concerning the injectable solutions. In the US pharmacopoeia, the solutions are defined in part <1151> referring to the injection (<1>) (referring to <788> according to USP 35 and specified in <788> according to USP 35 and in <787>, <788> and <790> USP 38 (from August 1, 2014), according to USP 38). In the European pharmacopoeia, the injectable solutions have to meet the criteria given in sections 2.9.19 and 2.9.20.

In an embodiment the invention relates to a composition, characterized in that it is a freeze-dried formulation.

In one embodiment the physician or the patient adds solvents and/or diluents prior to use.

In another embodiment the pharmaceutical formulation is a dried formulation (e.g. freeze-dried or spray-dried) ready for use without any prior dissolution.

By "dried form" is intended the liquid pharmaceutical composition or formulation is dried either by freeze-drying (i.e., lyophilization), spray-drying or air-drying.

In an embodiment the invention relates to a composition, characterized in that the pH is ranging from 3.5 to 4.4.

In an embodiment the invention relates to a composition, characterized in that the pH of the solution is comprised from 3.5 to 4.4.

In an embodiment the invention relates to a composition, characterized in that the pH of the solution is comprised from 3.7 to 4.2.

In an embodiment the invention relates to a composition, characterized in that the pH of the solution is about 4.0.

In an embodiment the invention relates to a composition, characterized in that the pH of the solution is ranging from 6.8 to 7.4

The invention also relates to a composition according to the invention which is intended to be used in an obesity treatment method, characterized in that it enables to decrease the food consumption. Obese persons being defined as persons having a BMI of 30 or higher.

The invention also relates to a composition according to the invention which is intended to be used in an overweight treatment method, characterized in that it enables to decrease the food consumption. Overweight people being defined as having a BMI from 25 to less than 30.

In an embodiment the composition according to the invention is intended to be used in an overweight or obesity treatment method for people having a BMI of 25 or higher.

In an embodiment the composition according to the invention is intended to be used in an overweight or obesity treatment method for people having a BMI of 27 or higher.

The invention also relates to a composition according to the invention which is intended to be used in method for the treatment of diabetes, characterized in that it enables to decrease the food consumption.

The invention also relates to a composition according to the invention which is intended to be used in an obesity treatment method, characterized in that it improves loss of weight or limit the gain of weight.

In an embodiment, the compositions according to the invention lead to a slow-down of the gastric emptying of at least 5 % inferior to an identical composition without the corresponding GLP-1 RA.

In one embodiment, the composition is characterized in that it comprises a concentration of pramlintide ranging from 0.5 to 10 mg/ml.

In one embodiment, the composition is characterized in that it comprises a concentration of pramlintide ranging from 0.6 to 6 mg/ml.

In one embodiment, the composition is characterized in that it comprises a concentration of pramlintide ranging from 0.9 to 4 mg/ml.

In one embodiment, the composition is characterized in that it comprises a concentration of pramlintide ranging from 1 to 4 mg/ml.

In one embodiment, the composition is characterized in that it comprises a concentration of pramlintide ranging from 1 to 3 mg/ml.

In one embodiment, the composition is characterized in that it comprises a concentration of pramlintide ranging from 1 to 2 mg/ml.

In one embodiment, the pramlintide concentration is 1 mg/ml.

In one embodiment, the pramlintide concentration is 1.5 mg/ml.

In one embodiment, the pramlintide concentration is 2 mg/ml.

In one embodiment, the pramlintide concentration is 2.5 mg/ml.

In one embodiment, the pramlintide concentration is 3 mg/ml.

In one embodiment, the pramlintide concentration is 3.5 mg/ml.

In one embodiment, the pramlintide concentration is 4 mg/ml.

In one embodiment, the pramlintide concentration is 4.5 mg/ml.

In one embodiment, the pramlintide concentration is 5 mg/ml.

In one embodiment, the composition is characterized in that it comprises a concentration of exenatide ranging from 20 to 500 µg/ml.

In one embodiment, the composition is characterized in that it comprises a concentration of exenatide ranging from 20 to 200 µg/ml

In one embodiment, the composition is characterized in that it comprises a concentration of exenatide ranging from 60 to 400 µg/ml.

In one embodiment, the composition is characterized in that it comprises a concentration of exenatide ranging from 80 to 300 µg/ml.

In one embodiment, the composition is characterized in that it comprises a concentration of exenatide ranging from 90 to 250 µg/ml.

In one embodiment, the composition is characterized in that it comprises a concentration of exenatide ranging from 100 to 200 µg/ml.

In one embodiment exenatide concentration is 50 µg/ml.

In one embodiment exenatide concentration is 75 µg/ml.

In one embodiment exenatide concentration is 100 µg/ml.

In one embodiment exenatide concentration is 125 µg/ml.

In one embodiment exenatide concentration is 150 µg/ml.

In one embodiment exenatide concentration is 175 µg/ml.

In one embodiment exenatide concentration is 200 µg/ml.

In one embodiment exenatide concentration is 250 µg/ml.

In one embodiment exenatide concentration is 300 µg/ml.

In one embodiment exenatide concentration is 350 µg/ml.

In one embodiment exenatide concentration is 400 µg/ml.

In one embodiment exenatide concentration is 450 µg/ml.

In one embodiment exenatide concentration is 500 µg/ml.

In one embodiment, the composition is characterized in that it comprises a concentration of lixisenatide ranging from 50 to 1250 µg/ml.

In one embodiment, the composition is characterized in that it comprises a concentration of lixisenatide ranging from 125 to 1000 µg/ml.

In one embodiment, the composition is characterized in that it comprises a concentration of lixisenatide ranging from 150 to 1000 µg/ml.

In one embodiment, the composition is characterized in that it comprises a concentration of lixisenatide ranging from 200 to 750 µg/ml.

In one embodiment, the composition is characterized in that it comprises a concentration of lixisenatide ranging from 225 to 650 µg/ml.

In one embodiment, the composition is characterized in that it comprises a concentration of lixisenatide ranging from 250 to 500 µg/ml.

In one embodiment lixisenatide concentration is 150 µg/ml.

In one embodiment lixisenatide concentration is 200 µg/ml.

In one embodiment lixisenatide concentration is 250 µg/ml.

In one embodiment lixisenatide concentration is 300 µg/ml.

In one embodiment lixisenatide concentration is 350 µg/ml.

In one embodiment lixisenatide concentration is 400 µg/ml.

In one embodiment lixisenatide concentration is 450 µg/ml.

In one embodiment lixisenatide concentration is 500 µg/ml.

In one embodiment lixisenatide concentration is 550 µg/ml.

In one embodiment lixisenatide concentration is 600 µg/ml.

In one embodiment lixisenatide concentration is 650 µg/ml.

In one embodiment lixisenatide concentration is 700 µg/ml.

In one embodiment lixisenatide concentration is 750 µg/ml.

In one embodiment lixisenatide concentration is 800 µg/ml.

In one embodiment, the composition is characterized in that ratio pramlintide / exenatide (weight/weight) is ranging from 2 to 50.

In one embodiment, the composition is characterized in that ratio pramlintide / exenatide (weight/weight) is ranging from 2 to 30.

In one embodiment, the composition is characterized in that ratio pramlintide / exenatide (weight/weight) is ranging from 3 to 25.

In one embodiment, the composition is characterized in that ratio pramlintide / exenatide (weight/weight) is ranging from 5 to 20.

In one embodiment, the composition is characterized in that ratio pramlintide / exenatide (weight/weight) is ranging from 8 to 15.

In one embodiment, the composition is characterized in that it comprises from 20 to 400 µg of Exenatide per mg of Pramlintide.

In one embodiment, the composition is characterized in that it comprises from 30 to 200 µg of Exenatide per mg of Pramlintide.

In one embodiment, the composition is characterized in that it comprises from 40 to 150 µg of Exenatide per mg of Pramlintide.

In one embodiment, the composition is characterized in that it comprises from 40 to 120 µg of Exenatide per mg of Pramlintide.

In one embodiment, the composition is characterized in that ratio pramlintide / lixisenatide (weight/weight) is ranging from 0.7 to 20.

In one embodiment, the composition is characterized in that ratio pramlintide / lixisenatide (weight/weight) is ranging from 0.7 to 15.

In one embodiment, the composition is characterized in that ratio pramlintide / lixisenatide (weight/weight) is ranging from 1.2 to 8.

In one embodiment, the composition is characterized in that ratio pramlintide / lixisenatide (weight/weight) is ranging from 1.8 to 7.

In one embodiment, the composition is characterized in that ratio pramlintide / lixisenatide (weight/weight) is ranging from 2.5 to 6.

In one embodiment, the composition is characterized in that it comprises from 50 to 1000 µg of lixisenatide per mg of Pramlintide.

In one embodiment, the composition is characterized in that it comprises from 75 to 500 µg of lixisenatide per mg of Pramlintide.

In one embodiment, the composition is characterized in that it comprises from 100 to 375 µg of lixisenatide per mg of Pramlintide.

In one embodiment, the composition is characterized in that it comprises from 100 to 300 µg of lixisenatide per mg of Pramlintide.

In an embodiment, the compositions according to the invention moreover include buffers.

In an embodiment, the compositions according the invention include a buffer selected from the group consisting of a sodium acetate buffer and Tris buffer.

In one embodiment, the composition is characterized in that it comprises a sodium acetate buffer.

In one embodiment, the composition is characterized in that it comprises a Tris buffer

In an embodiment, the compositions according the invention moreover include preservatives.

In an embodiment, the preservatives are selected from the group consisting of m-cresol and phenol, alone or in a mixture.

In an embodiment, the concentration of preservatives is from 10 to 50 mM.

In an embodiment, the concentration of preservatives is from 10 to 40 mM.

In an embodiment, the compositions according to the invention moreover include a surfactant.

In an embodiment, the surfactant is selected from the group consisting of Poloxamer 188, Tween® 20, also referred to as Polysorbate 20, and Tween® 80, also referred to as Polysorbate 80.

In an embodiment, the Tween® 20 concentration varies from 5 to 50 µg/mL.

In an embodiment, the Tween® 20 concentration varies from 5 to 25 µg/mL.

In an embodiment, the Tween® 20 concentration is 10 µg/mL

In one embodiment, the composition is characterized in that the concentration of Poloxamer 188 is ranging from 5 to 50 µg/ml.

In one embodiment, the composition is characterized in that the concentration of Poloxamer 188 is ranging from 5 to 25 µg/ml.

In one embodiment, the composition is characterized in that the concentration of Poloxamer 188 is 10 µ/mL.

The compositions according to the invention can moreover include additives such as tonicity agents.

In an embodiment, the tonicity agents are selected from the group consisting of glycerol, sodium chloride, mannitol and glycine.

In an embodiment, the compositions according to the invention moreover include an antioxidant.

In an embodiment, the antioxidant is methionine.

In an embodiment, the composition according to the invention is characterized in that it does not comprise insulin.

In an embodiment, the composition according to the invention is characterized in that it does not comprise human insulin.

In an embodiment, the composition according to the invention is characterized in that it does not comprise insulin chosen amongst human insulin analogs.

In an embodiment, the composition according to the invention is characterized in that it does not comprise human insulin analogs chosen amongst insulin aspart (NovoLog® from NOVO NORDISK), insulin lispro (Humalog® from ELI LILLY) and glulisine insulin (Apidra® from SANOFI).

In an embodiment, the composition according to the invention is characterized in that it does not comprise human insulin analogs chosen amongst insulin comprising at least the A21G mutation.

In an embodiment, the composition according to the invention is characterized in that it does not comprise human insulin analogs are chosen amongst insulin analogs A21G, B28D, desB30 and A21G, B28E, desB30 described in application WO2007104786 entirely included herein by reference.

In an embodiment, the composition according to the invention is characterized in that it does not comprise the human insulin analogs comprising only the mutation A21G.

The chain A sequence is SEQ ID NO :4 GIVEQCCTSICSLYQLENYCG and the chain B sequence is SEQ ID NO :5 FVNQHLCGSHLVEALYLVCGERGFFYTPKT.

In an embodiment, the composition according to the invention is characterized in that it does not comprise insulin glargine.

In an embodiment, the composition according to the invention is characterized in that it does not comprise glucagon or glucagon analogs.

Composition according to the invention, characterized in that it does not comprise co-polyaminoacids bearing carboxylate charges and hydrophobic radicals.

In an embodiment, the hydrophobic radicals are chosen amongst those as described in the application US2019274954A1 entirely included herein by reference.

In an embodiment said hydrophobic radicals are chosen amongst the radicals according to formula X as defined below: 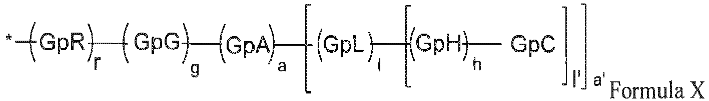 wherein
- GpR is chosen among the radicals according to formulas VII, VII' or VII": 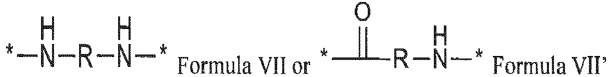 or 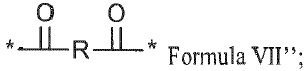
- GpG and GpH identical or different are chosen among the radicals according to formulas XI or XI': 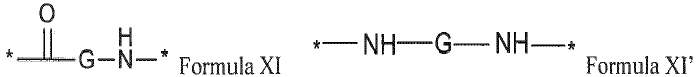
- GpA is chosen among the radicals according to formula VIII 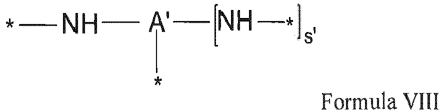 wherein A' is chosen among the radicals according to VIII', VIII" or VIII''' 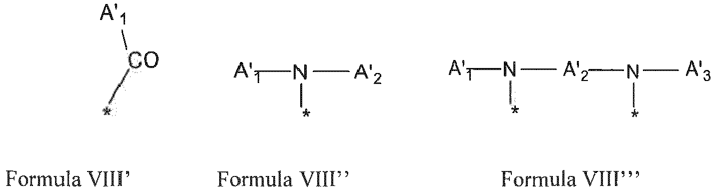
- -GpL is chosen among the radicals according to formula XII 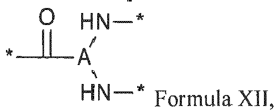
- GpC is a radical according to formula IX: 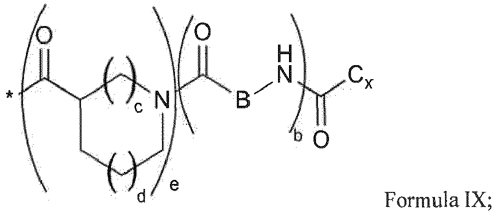
- the * indicate the binding sites of the different groups bound by amide functions;
- a is an integer equal to 0 or to 1 and a' = 1 if a = 0 and a' = 1, 2 or 3 if a = 1;
- a' is an integer equal to 1, to 2 or to 3;
- b is an integer equal to 0 or to 1;
- c is an integer equal to 0 or to 1, and if c is equal to 0 then d is equal to 1 or to 2;
- d is an integer equal to 0, to 1 or to 2;
- e is an integer equal to 0 or to 1;
- g is an integer equal to 0, to 1, to 2, to 3 to 4 to 5 or to 6;
- h is an integer equal to 0, to 1, to 2, to 3 to 4 to 5 or to 6;
- l is an integer equal to 0 or 1 and l' = 1 if l = 0 and l' = 2 if l = 1;
- l' is an integer equal to 1 or to 2;
- r is an integer equal to 0, 1 or to 2, and
- s' is an integer equal to 0 or 1;
- A'₁, A'₂, and A'₃ identical or different are linear or branched alkyl radicals comprising from 1 to 8 carbon atoms, and optionally substituted by a radical from a saturated, unsaturated or aromatic ring;
- B is a radical chosen in the group consisting of a non-substituted ether or polyether radical comprising from 4 to 14 carbon atoms and from 1 to 5 oxygen atoms or a linear or branched alkyl radical, optionally comprising an aromatic nucleus, comprising from 1 to 9 carbon atoms;
- Cₓ is a radical chosen in the group consisting of a linear or branched monovalent alkyl radical, optionally comprising a cyclic part, wherein x indicates the number of carbon atoms and 6≤x≤25:
   ▪ When the hydrophobic radical -Hy bears 1 -GpC, then 9 ≤ x ≤ 25,
   ▪ When the hydrophobic radical -Hy bears 2 -GpC, then 9 ≤ x ≤ 15,
   ▪ When the hydrophobic radical -Hy bears 3 -GpC, then 7 ≤ x ≤ 13,
   ▪ When the hydrophobic radical -Hy bears 4 -GpC, then 7 ≤ x ≤ 11,
   ▪ When the hydrophobic radical -Hy bears at least 5 -GpC, then 6 ≤ x ≤ 11;
- G is a linear or branched divalent alkyl radical of 1 to 8 carbon atoms, said alkyl radical bearing one or a plurality of free carboxylic acid function(s),
- R is a radical chosen in the group consisting of a linear or branched, divalent alkyl radical comprising from 1 to 12 carbon atoms, a linear or branched, divalent alkyl radical comprising from 1 to 12 carbon atoms bearing one or a plurality of functions -CONH₂ or a non-substituted ether or polyether radical comprising from 4 to 14 carbon atoms and from 1 to 5 oxygen atoms
- the hydrophobic radical(s) -Hy according to formula X being bound to the PLG:
   ∘ via a covalent bond between a carbonyl of the hydrophobic radical -Hy and a nitrogen atom borne by the PLG thus forming an amide function obtained from the reaction of an amine function borne by the PLG and an acid function borne by the precursor Hy' of the hydrophobic radical -Hy and/or
   ∘ via a covalent bond between a nitrogen atom of the hydrophobic radical -Hy and a carbonyl borne by the PLG thus forming an amide function obtained from the reaction of an amine function of the precursor Hy' of the hydrophobic radical -Hy and an acid function borne by the PLG;
- the ratio M between the number of hydrophobic radicals and the number of glutamic or aspartic units being comprised from 0 < M ≤ 0.5;
- when a plurality of hydrophobic radicals are borne by a co-polyamino acid then they are identical or different;
- the degree of polymerization DP in glutamic or aspartic units for the PLG chains is comprised from 5 to 250;
- the free acid functions being in the form of alkali cation salt chosen in the group consisting of Na⁺ and K⁺.

In an embodiment composition according to the invention is characterized in that it does not comprise a co-polyamino acid bearing carboxylate charges and hydrophobic radicals as described in the application US20190328842A1 entirely included herein by reference.

In an embodiment composition according to the invention is characterized in that it does not comprise a co-polyamino acid bearing carboxylate charges and hydrophobic radicals Hy, said co-polyamino acid being constituted of glutamic or aspartic units and said hydrophobic radicals -Hy chosen among the radicals according to formula X₁ as defined below: 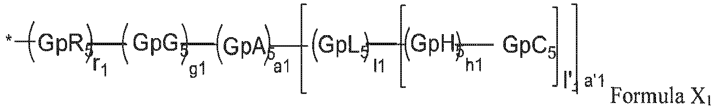 In which
- GpR₅ is chosen among the radicals according to formulas VII₁, VII'₁ or VII"₁: 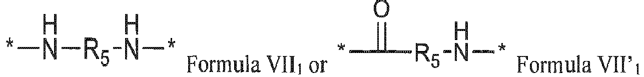 or 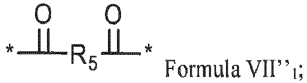
- Identical or different GpG₅ and GpH₅ are chosen among the radicals according to formulas XI₁ or XI'₁; 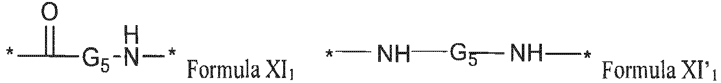
   - GpA₅ is chosen among the radicals according to formula VIII₁ 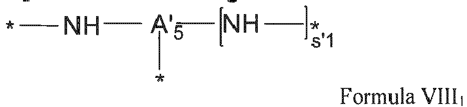 In which A'₅ is chosen among the radicals according to formulas VIII'₁, VIII"₁ or VIII'''₁ 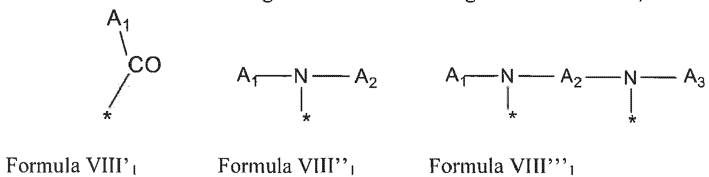
   - GpL₅ is chosen among the radicals according to formula XII₁ 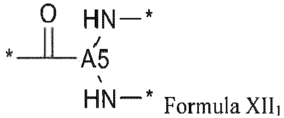
   - GpC₅ is a radical according to formula IX₁: 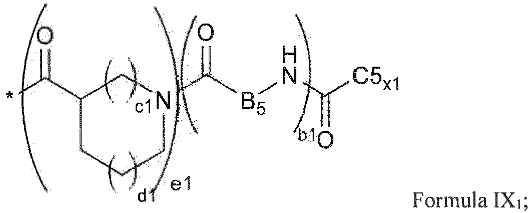
- * indicate the attachment sites of the different groups bound by amide functions;
- a₁ is an integer equal to 0 or to 1 and a'₁ = 1 if a₁ = 0 and a'₁ = 1, 2 or 3 if a₁ =1;
- a'₁ is an integer equal to 1, to 2 or to 3;
- b₁ is an integer equal to 0 or to 1;
- c₁ is an integer equal to 0 or to 1, and if c₁ is equal to 0, then d₁ is equal to 1 or to 2;
- d₁ is an integer equal to 0, to 1 or to 2;
- e₁ is an integer equal to 0 or to 1;
- g₁ is an integer equal to 0, to 1, to 2, to 3, to 4, to 5 or to 6;
- h₁ is an integer equal to 0, to 1, to 2, to 3, to 4, to 5 or to 6, and at least one of g₁, h₁ or l₁ is different from 0;
- l₁ is an integer equal to 0 or to 1 and l'₁ = 1 if l₁ = 0 and l'₁ = 2 if l₁ =1;
- r₁ is an integer equal to 0, 1 or to 2, and
- s'₁ is an integer equal to 0 or to 1;
- And if e₁ is different from 0, then at least one of gi, h₁ or l₁ is different from 0;
- And if a₁ = 0, then l₁ = 0;
- A5, A₁, A₂ and A₃ identical or different, are linear or branched alkyl radicals comprising from 1 to 8 carbon atoms and, optionally, substituted by a radical from a saturated, unsaturated or aromatic ring;
- B₅ is a radical ether or polyether, unsubstituted, comprising from 4 to 14 carbon atoms and 1 to 5 oxygen atoms, or a linear or branched alkyl radical, optionally comprising an aromatic ring, comprising from 1 to 9 carbon atoms.
- C5ₓ₁ is a monovalent, linear or branched, alkyl radical optionally comprising a cyclic part, in which x₁ indicates the number of carbon atoms, and:
   ▪ When the hydrophobic radical -Hy bears 1 -GpC5, then 9 ≤ x₁ ≤ 25,
   ▪ When the hydrophobic radical -Hy bears 2 -GpC5, then 9 ≤ x₁ ≤ 15,
   ▪ When the hydrophobic radical -Hy bears 3 -GpC5, then 7 ≤ x₁ ≤ 13,
   ▪ When the hydrophobic radical -Hy bears 4 -GpC5, then 7 ≤ x₁ ≤ 11,
   ▪ When the hydrophobic radical -Hy bears at least 5 -GpC5, then 6 ≤ x₁ ≤ 11,
- G5 is a linear or branched divalent alkyl radical of 1 to 8 carbon atoms, said alkyl radical bearing one or more free carboxylic acid functions.
- R5 is a radical chosen from the group consisting of a divalent, linear or branched alkyl radical comprising from 1 to 12 carbon atoms, a divalent, linear or branched alkyl radical comprising from 1 to 12 carbon atoms bearing one or more -CONH₂ functions or an unsubstituted ether or polyether radical comprising from 4 to 14 carbon atoms and 1 to 5 oxygen atoms.
- The hydrophobic radicals -Hy according to formula X₁ being bound to the PLG:
   ∘ via a covalent bond between a carbonyl of the hydrophobic radical -Hy and a nitrogen atom borne by the PLG, thus forming an amide function resulting from the reaction of an amine function borne by the PLG and an acid function borne by the precursor -Hy' of the hydrophobic radical -Hy, and
   ∘ via a covalent bond between a nitrogen atom of the hydrophobic radical -Hy and a carbonyl borne by the PLG, thus forming an amide function resulting from the reaction of an amine function of the precursor -Hy' of the hydrophobic radical -Hy and an acid function borne by the PLG.
- The ratio M between the number of hydrophobic radicals and the number of glutamic or aspartic unites being between 0 < M ≤ 0.5;
- When several hydrophobic radicals are borne by a co-polyamino acid, then they are identical or different,
- The degree of polymerization DP in glutamic or aspartic units for the PLG chains is comprised from 5 to 250;
- Free carboxylic acids being in the form of an alkaline cation salt chosen from the group consisting of Na⁺ and K⁺.

In an embodiment composition according to the invention is characterized in that it does not comprise a co-polyamino acid bearing carboxylate charges and hydrophobic radicals as described in the application US2019274954A1 entirely included herein by reference.

In an embodiment composition according to the invention is characterized in that it does not comprise a co-polyamino acid bearing carboxylate charges carboxylates and at least one hydrophobic radical -Hy according to formula X as defined above, said co-polyamino acid being chosen among the co-polyamino acids according to formula I₂:

[Q(PLG)ₖ₂][Hy]ⱼ₂[Hy]_{j'2} Formula I₂

Wherein:
- J₂ ≥ 1; 0 ≤ j'₂ ≤ n'1 and j₂ + j'₂ ≥ 1 and k₂ ≥ 2
- said co-polyamino acid according to formula I₂ bearing at least one hydrophobic radical -Hy, carboxylate charges and consisting of at least two chains of glutamic or aspartic units PLG bound together by an at least divalent linear or branched radical or spacer Q[-*]ₖ₂ consisting of an alkyl chain comprising one or a plurality of heteroatoms chosen in the group consisting of nitrogen and oxygen atoms and/or bearing one or a plurality of heteroatoms consisting of nitrogen and oxygen radicals and/or radicals bearing one or a plurality of heteroatoms consisting of nitrogen and oxygen atoms and/or carboxyl functions.
- said radical or spacer Q[-*]k₂ being bound to at least two glutamic or aspartic unit chains PLG by an amide function and,
- said amide bonds binding said radical or spacer Q[-*]k₂ bound to said at least two chains of glutamic or aspartic units result from the reaction between an amine function and an acid function respectively borne either by the precursor Q' of the radical or spacer Q[-*]k₂ or by a glutamic or aspartic unit,
- said hydrophobic radical -Hy being bound either to a terminal "amino acid" unit and then j₂ ≥ 1, or to a carboxyl function borne by one of the chains of the glutamic or aspartic units PLG and then j'₂ = n'1 and n'1 is the mean number of monomeric units bearing a hydrophobic radical -Hy.

In an embodiment composition according to the invention is characterized in that it does not comprise a co-polyamino acid bearing carboxylate charges and hydrophobic radicals as described in the applications US20180193421A1 and US2019275108A1 entirely included herein by reference.

In an embodiment composition according to the invention is characterized in that it does not comprise a co-polyamino acid bearing carboxylate charges and hydrophobic radicals Hy, said co-polyamino acid consisting of glutamic or aspartic units and said hydrophobic radicals Hy having the following formula I₃: 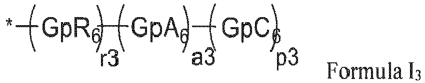 in which
- GpR₆ is a radical of formula II₃ or II'₃ or II"₃: 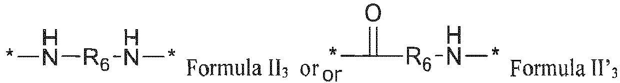 or 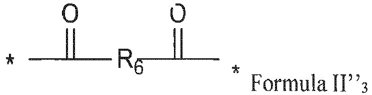
- GpA₆ is a radical of formula III₃ or III'₃: 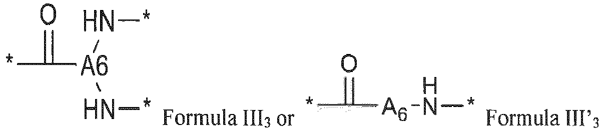
- GpC₆ is a radical of formula IV₃: 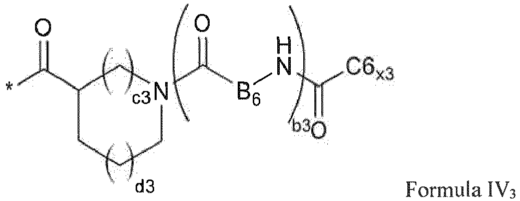
- the * indicate the sites of attachment of the different groups;
- a₃ is a whole number equal to 0 or to 1;
- b₃ is a whole number equal to 0 or to 1;
- p₃ is a whole number equal to 1 or 2 and
   o if p₃ is equal to 1 then a₃ is equal to 0 or to 1 and GpA₆ is a radical of formula III'₃, and
   o if p₃ is equal to 2 then a₃ is equal to 1, and GpA₆ is a radical of formula III₃;
- c₃ is a whole number equal to 0 or to 1, and if c₃ is equal to 0 then d₃ is equal to 1 or to 2;
- d₃ is a whole number equal to 0, to 1 or to 2;
- r₃ is a whole number equal to 0 or to 1, and
   ∘ if r₃ is equal to 0 then the hydrophobic radical of formula I₃ is attached to the co-polyamino acid via a covalent bond between a carbonyl of the hydrophobic radical and a nitrogen atom in N-terminal position of the co-polyamino acid, thus forming an amide function originating from the reaction of an amine function in N-terminal position of the precursor of the co-polyamino acid and an acid function borne by the precursor of the hydrophobic radical, and
   ∘ if r₃ is equal to 1 then the hydrophobic radical of formula I₃ is attached to the co-polyamino acid:
      ▪ via a covalent bond between a nitrogen atom of the hydrophobic radial and a carbonyl of the copolyamino acid, thus forming an amide function originating from the reaction of an amine function of the precursor of the hydrophobic radical and an acid function borne by the precursor of the co-polyamino acid, or
      ▪ via a covalent bond between a carbonyl of the hydrophobic radical and a nitrogen atom in N-terminal position of the co-polyamino acid, thus forming an amide function originating from the reaction of an acid function of the precursor of the hydrophobic radical and an amine function in N-terminal position borne by the precursor of the co-polyamino acid;
- R₆ is a radical selected from the group consisting of:
   ∘ a linear or branched divalent alkyl radical comprising 2 to 12 carbon atoms if GpR₆ is a radical of formula II₃ or 1 to 11 carbon atoms if GpR₆ is a radical of formula II'₃ or II"₃;
   ∘ a linear or branched divalent alkyl radical comprising 2 to 11 carbon atoms if GpR₆ is a radical of formula II₃ or 1 to 11 carbon atoms if GpR₆ is a radical of formula II'₃ or II"₃, 1 to 11 carbon atoms, said alkyl radical bearing one or more -CONH2 functions, and
   ∘ an unsubstituted ether or polyether radical comprising 4 to 14 carbon atoms and 1 to 5 oxygen atoms;
- A6 is a linear or branched alkyl radical comprising 1 to 6 carbon atoms;
- B6 is a linear or branched alkyl radical, optionally comprising an aromatic ring, comprising 1 to 9 carbon atoms;
- C6x₃ is a linear or branched monovalent alkyl radical, in which x₃ indicates the number of carbon atoms and:
   ∘ if p₃ is equal to 1, x₃ is from 11 to 25 (11 ≤ x₃ ≤ 25):
   ∘ if p₃ is equal to 2, x₃ is from 9 to 15 (9 ≤ x₃ ≤ 15),
- the ratio i between the number of hydrophobic radicals and the number of glutamic or aspartic units being from 0 to 0.5 (0 < i ≤ 0.5);
- when several hydrophobic radicals are borne by a co-polyamino acid, then they are identical or different,
- the degree of polymerization DP of glutamic or aspartic units is from 5 to 250;
- the free acid functions being in the form of a salt of an alkali cation selected from the group consisting of Na+ and K+;
characterized in that the composition does not comprise a basal insulin of which the isoelectric point pI is from 5.8 to 8.5.

In an embodiment composition according to the invention is characterized in that it does not comprise an amphiphilic compound comprising a hydrophilic backbone substituted by hydrophobic radicals as described in the application PCT/EP/2019/070953 entirely included herein by reference.

In an embodiment composition according to the invention is characterized in that it does not comprise an amphiphilic compound comprising a hydrophilic backbone substituted hydrophobic radicals Hy, said hydrophobic radicals being chosen from formula 14:

*-(GpR₇)ᵣ₄-(GpI₇)ᵢ₄-[(GpR₇)_{r'4}-(GpI₇)_{i'4}]ₜ₄-GpC₇ Formula I₄

Wherein :
- GpI₇ is a divalent radical, said radical comprising at least one imidazol radical Im of formula III₄ : 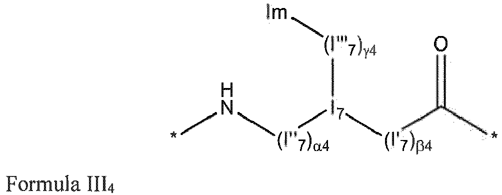
- GpR₇ is a radical of formulas II₄, II'₄ or II''₄ : 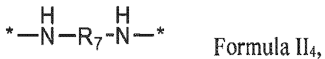 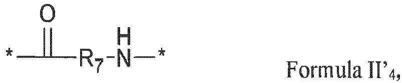 or 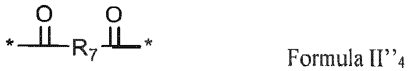
- GpC₇ is a radical of formula IV₄ : 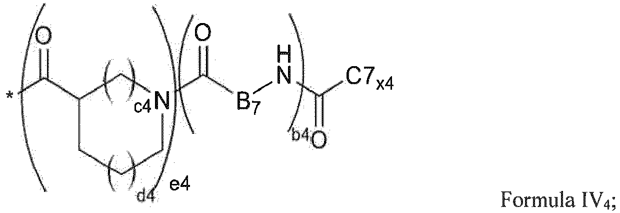 the * indicate the sites of attachment of the hydrophobic radical -Hy to the hydrophilic backbone HB or the above radicals (I₄, II₄, II'₄, II''₄, III₄ and IV₄) to each other by amide functions;
- a₄, b₄ and g₄ are identical or different integers equal to 0 or 1 ;
- b₄ is a integer equal to 0 or 1 ;
- c₄ is a integer equal to 0 or 1 ;
- d₄ is a integer equal to 0, 1 or 2 ; and if c₄ equal to 0, then d₄ equal to 1 or 2;
- e₄ is a integer equal to 0 or 1 ;
- i₄ and i'₄ are identical or different integers less than or equal to 6 and i₄ + i'₄ is greater than or equal to 1 and less than or equal to 6, 1 ≤ i₄ + i'₄ ≤6,
- r₄ and r'₄ are integers equal to 0, 1, 2 or 3 ;
- if r₄ is equal to 0 then the hydrophobic radical of formula I₄ is linked to the hydrophilic backbone HB via a covalent bond between a carbonyl of the hydrophobic radical and a nitrogen atom of the hydrophilic backbone HB, thus forming an amide function resulting from the reaction of an amine function of the precursor of the hydrophilic backbone HB and a function acid carried by the precursor of the hydrophobic radical, and
- if r₄ is equal to 1, 2 or 3 then the hydrophobic radical -Hy of formula I₄ is linked to the hydrophilic backbone HB:
   ∘ via a covalent bond between a nitrogen atom of the hydrophobic radical and a carbonyl of the hydrophilic backbone HB, thus forming an amide function resulting from the reaction of an amine function of the precursor of the hydrophobic radical and an acid function carried by the precursor of the hydrophilic backbone HB or
   ∘ via a covalent bond between a carbonyl of hydrophobic radical and a nitrogen atom of the hydrophilic backbone HB, thus forming an amide function resulting from the reaction of an acid function of the precursor of the hydrophobic radical and an amine function of the precursor of the hydrophilic backbone HB;
- t4 is a integer equal to 0 or 1 ;
- B7 is a linear or branched alkyl radical, optionally comprising an aromatic ring, comprising from 1 to 9 carbon atoms or an unsubstituted ether or polyether radical comprising from 4 to 14 carbon atoms and from 1 to 5 oxygen atoms;
- C7ₓ₄ is a linear or branched monovalent alkyl radical, optionally comprising a cyclic part, in which x₄ indicates the number of carbon atoms and 11≤x₄≤25 ;
- I'₄, I"₄ and I'''₄, identical or different, are divalent radicals, chosen from the group consisting of an alkyl radical, linear or branched comprising from 1 to 12 carbon atoms,
- I₄ is a trivalent radical, chosen from the group consisting of a linear or branched alkyl radical comprising from 1 to 12 carbon atoms;
- Im is a imidazolyl radical,
- R₇ is a radical chosen from the group consisting of a divalent, linear or branched alkyl radical comprising from 1 to 12 carbon atoms, a branched alkyl radical from 1 to 8 carbon atoms, said alkyl radical carrying one or more free carboxylic acid function(s), a divalent, linear or branched alkyl radical comprising from 1 to 12 carbon atoms carrying one or more -CONH2 function(s) or an unsubstituted ether or polyether radical comprising from 4 to 14 carbon atoms and from 1 to 5 atoms oxygen, said free carboxylic acid functions being in the form of alkaline cation salts chosen from the group consisting of Na + and K +, and
when several hydrophobic radicals are carried by a hydrophilic HB backbone then they are identical or different.

The invention also concerns composition for use in the method for the treatment of obesity.

The invention also concerns composition for use in the method for the treatment of overweight.

The invention also concerns composition for use in the method for the treatment of diabetes, in particular type 2 diabetes.

In an embodiment the method of treatment concerns animals.

In another embodiment the method of treatment concerns mammals.

In another embodiment the method of treatment concerns mammals pets, such as dogs and cats.

In another embodiment the method of treatment concerns human beings.

In one embodiment, the composition according to the invention is for use in the method for the treatment of obesity, overweight and/or diabetes, in particular type 2 diabetes, characterized in that it reduces the slowing effect of gastric emptying of amylin RA as compared to the same composition without GLP-1 RA.

In one embodiment, the composition according to the invention is for use in the method for the treatment of obesity, overweight and/or diabetes, in particular type 2 diabetes, characterized in that it reduces the slowing effect on gastric emptying of amylin RA as compared to the same composition without GLP-1 RA by at least 5%.

In one embodiment, the composition according to the invention is for use in the method for the treatment of obesity, overweight and/or diabetes, in particular type 2 diabetes, characterized in that it reduces the slowing effect on gastric emptying of amylin RA as compared to the same composition without GLP-1 RA by at least 10%.

In one embodiment, the composition according to the invention is for use in the method for the treatment of obesity, overweight and/or diabetes, in particular type 2 diabetes, characterized in that it reduces the slowing effect on gastric emptying of amylin RA as compared to the same composition without GLP-1 RA by at least 20%.

In one embodiment, the composition according to the invention is for use in the method for the treatment of obesity, overweight and/or diabetes, in particular type 2 diabetes, characterized in that it reduces the slowing effect on gastric emptying of amylin RA as compared to the same composition without GLP-1 RA by at least 30%.

In one embodiment, the composition according to the invention is for use in the method for the treatment of obesity, overweight and/or diabetes, in particular type 2 diabetes, characterized in that it reduces the slowing effect on gastric emptying of amylin RA as compared to the same composition without GLP-1 RA by at least 40%.

In one embodiment, the composition according to the invention is for use in the method for the treatment of obesity, overweight and/or diabetes, in particular type 2 diabetes, characterized in that it reduces at least one adverse effect as compared to the same composition without GLP-1 RA.

In one embodiment, the composition according to the invention for the treatment of obesity, overweight and/or diabetes, in particular Type 2 Diabetes, is characterized in that the daily dose of pramlintide is ranging from 150 to 2000 µg/day.

In one embodiment, the composition according to the invention for the treatment of obesity and/or diabetes, in particular Type 2 Diabetes, is characterized in that the daily dose of pramlintide is ranging from 250 to 1800 µg/day.

In one embodiment, the composition according to the invention for the treatment of obesity and/or diabetes, in particular Type 2 Diabetes, is characterized in that the daily dose of pramlintide is ranging from 300 to 1500 µg/day.

In one embodiment, the composition according to the invention for the treatment of obesity and/or diabetes, in particular Type 2 Diabetes, is characterized in that the daily dose of pramlintide is ranging from 350 to 1200 µg/day.

In one embodiment, the composition according to the invention for the treatment of obesity and/or diabetes, in particular Type 2 Diabetes, is characterized in that the daily dose of pramlintide is ranging from 400 to 1000 µg/day.

In one embodiment, the composition according to the invention for the treatment of obesity and/or diabetes, in particular Type 2 Diabetes, is characterized in that the daily dose of pramlintide is ranging from 400 to 800 µg/day.

Compositions according to any of the preceding claims, characterized in that the daily dose of exenatide is ranging from 10 to 250 µg.

In one embodiment, the composition according to the invention, is characterized in that the daily dose of exenatide is ranging from 20 to 200 µg.

Compositions according to any of the preceding claims, characterized in that the daily dose of exenatide is ranging from 25 to 180 µg.

Compositions according to any of the preceding claims, characterized in that the daily dose of exenatide is ranging from 30 to 160 µg.

Compositions according to any of the preceding claims, characterized in that the daily dose of exenatide is ranging from 35 to 140 µg.

Compositions according to any of the preceding claims, characterized in that the daily dose of exenatide is ranging from 40 to 120 µg.

Compositions according to any of the preceding claims, characterized in that the daily dose of lixisenatide is ranging from 25 to 600 µg.

In one embodiment, the composition according to the invention, is characterized in that the daily dose of lixisenatide is ranging from 50 to 500 µg.

Compositions according to any of the preceding claims, characterized in that the daily dose of lisxenatide is ranging from 65 to 450 µg.

Compositions according to any of the preceding claims, characterized in that the daily dose of lisxenatide is ranging from 75 to 400 µg.

Compositions according to any of the preceding claims, characterized in that the daily dose of lixisenatide is ranging from 85 to 350 µg.

Compositions according to any of the preceding claims, characterized in that the daily dose of lixisenatide is ranging from 95 to 300 µg.

The invention also concerns a container comprising a pharmaceutical formulation according to the invention.

In one embodiment the container is a cartridge.

In one embodiment the container is a vial.

The invention also concerns an injection or delivery device comprising a pharmaceutical formulation according to the invention.

In one embodiment, the injection or delivery device comprises a pharmaceutical formulation according to the invention.

In one embodiment, the injection or delivery device is a pump.

In one embodiment, the injection or delivery device is characterized in that it delivers the pharmaceutical formulation continuously.

In one embodiment, the injection or delivery device is characterized in that it delivers microboluses of the pharmaceutical formulation.

In one embodiment, the injection or delivery device is characterized in that it is a patch pump

In one embodiment, the injection or delivery device is characterized in that it isan osmotic pump.

Composition according to the invention, characterized in that it comprises 0.6 mg/mL of pramlintide and 24 µg/mL of exenatide.

Composition according to the invention, characterized in that it comprises 0.6 mg/mL of pramlintide and 36 µg/mL of exenatide.

Composition according to the invention, characterized in that it comprises 0.6 mg/mL of pramlintide and 48 µg/mL of exenatide.

Composition according to the invention, characterized in that it comprises 0.6 mg/mL of pramlintide and 60 µg/mL.

Composition according to the invention, characterized in that it comprises 0.6 mg/mL of pramlintide and 72 µg/mL of exenatide.

Composition according to the invention, characterized in that it comprises 1.00 mg/mL of pramlintide and 40 µg/mL of exenatide.

Composition according to the invention, characterized in that it comprises 1.00 mg/mL of pramlintide and 60 µg/mL of exenatide.

Composition according to the invention, characterized in that it comprises 1.00 mg/mL of pramlintide and 80 µg/mL of exenatide

Composition according to the invention, characterized in that it comprises 1.00 mg/mL of pramlintide and 100 µg/mL of exenatide.

Composition according to the invention, characterized in that it comprises 1.00 mg/mL of pramlintide and 120 µg/mL of exenatide.

Composition according to the invention, characterized in that it comprises 2.00 mg/mL of pramlintide and 80 µg/mL of exenatide.

Composition according to the invention, characterized in that it comprises 2.00 mg/mL of pramlintide and 120 µg/mL of exenatide.

Composition according to the invention, characterized in that it comprises 2.00 mg/mL of pramlintide and 160 µg/mL of exenatide.

Composition according to the invention, characterized in that it comprises 2.00 mg/mL of pramlintide and 200 µg/mL of exenatide.

Composition according to the invention, characterized in that it comprises 2.00 mg/mL of pramlintide and 240 µg/mL of exenatide.

Composition according to the invention, characterized in that it comprises 3.00 mg/mL of pramlintide and 120 µg/mL of exenatide.

Composition according to the invention, characterized in that it comprises 3.00 mg/mL of pramlintide and 180 µg/mL of exenatide.

Composition according to the invention, characterized in that it comprises 3.00 mg/mL of pramlintide and 240 µg/mL of exenatide.

Composition according to the invention, characterized in that it comprises 3.00 mg/mL of pramlintide and 300 µg/mL of exenatide.

Composition according to the invention, characterized in that it comprises 3.00 mg/mL of pramlintide and 360 µg/mL of exenatide.

Composition according to the invention, characterized in that it comprises 4.00 mg/mL of pramlintide and 160 µg/mL of exenatide.

Composition according to the invention, characterized in that it comprises 4.00 mg/mL of pramlintide and 240 µg/mL of exenatide.

Composition according to the invention, characterized in that it comprises 4.00 mg/mL of pramlintide and 320 µg/mL of exenatide.

Composition according to the invention, characterized in that it comprises 4.00 mg/mL of pramlintide and 400 µg/mL of exenatide.

Composition according to the invention, characterized in that it comprises 4.00 mg/mL of pramlintide and 460 µg/mL of exenatide.

Composition according to the invention, characterized in that it comprises 0.6 mg/mL of pramlintide, 24 µg/mL of exenatide and m-cresol (20 mM), Acetate buffer (20 mM), Mannitol (250 mM).

Composition according to the invention, characterized in that it comprises 0.6 mg/mL of pramlintide, 36 µg/mL of exenatide and m-cresol (20 mM), Acetate buffer (20 mM), Mannitol (250 mM).

Composition according to the invention, characterized in that it comprises 0.6 mg/mL of pramlintide, 48 µg/mL of exenatide and m-cresol (20 mM), Acetate buffer (20 mM), Mannitol (250 mM).

Composition according to the invention, characterized in that it comprises 0.6 mg/mL of pramlintide, 60 µg/mL of exenatide and m-cresol (20 mM), Acetate buffer (20 mM), Mannitol (250 mM).

Composition according to the invention, characterized in that it comprises 0.6 mg/mL of pramlintide, 72 µg/mL of exenatide and m-cresol (20 mM), Acetate buffer (20 mM), Mannitol (250 mM).

Composition according to the invention, characterized in that it comprises 1.00 mg/mL of pramlintide, 40 µg/mL of exenatide and m-cresol (20 mM), Acetate buffer (20 mM), Mannitol (250 mM).

Composition according to the invention, characterized in that it comprises 1.00 mg/mL of pramlintide, 60 µg/mL of exenatide and m-cresol (20 mM), Acetate buffer (20 mM), Mannitol (250 mM).

Composition according to the invention, characterized in that it comprises 1.00 mg/mL of pramlintide, 80 µg/mL of exenatide and m-cresol (20 mM), Acetate buffer (20 mM), Mannitol (250 mM).

Composition according to the invention, characterized in that it comprises 1.00 mg/mL of pramlintide, 100 µg/mL of exenatide and m-cresol (20 mM), Acetate buffer (20 mM), Mannitol (250 mM).

Composition according to the invention, characterized in that it comprises 1.00 mg/mL of pramlintide, 120 µg/mL of exenatide and m-cresol (20 mM), Acetate buffer (20 mM), Mannitol (250 mM).

Composition according to the invention, characterized in that it comprises 2.00 mg/mL of pramlintide, 80 µg/mL of exenatide and m-cresol (20 mM), Acetate buffer (20 mM), Mannitol (250 mM).

Composition according to the invention, characterized in that it comprises 2.00 mg/mL of pramlintide, 120 µg/mL of exenatide and m-cresol (20 mM), Acetate buffer (20 mM), Mannitol (250 mM).

Composition according to the invention, characterized in that it comprises 2.00 mg/mL of pramlintide, 160 µg/mL of exenatide and m-cresol (20 mM), Acetate buffer (20 mM), Mannitol (250 mM).

Composition according to the invention, characterized in that it comprises 2.00 mg/mL of pramlintide, 200 µg/mL of exenatide and m-cresol (20 mM), Acetate buffer (20 mM), Mannitol (250 mM).

Composition according to the invention, characterized in that it comprises 2.00 mg/mL of pramlintide, 240 µg/mL of exenatide and m-cresol (20 mM), Acetate buffer (20 mM), Mannitol (250 mM).

Composition according to the invention, characterized in that it comprises 3.00 mg/mL of pramlintide, 120 µg/mL of exenatide and m-cresol (20 mM), Acetate buffer (20 mM), Mannitol (250 mM).

Composition according to the invention, characterized in that it comprises 3.00 mg/mL of pramlintide, 180 µg/mL of exenatide and m-cresol (20 mM), Acetate buffer (20 mM), Mannitol (250 mM).

Composition according to the invention, characterized in that it comprises 3.00 mg/mL of pramlintide, 240 µg/mL of exenatide and m-cresol (20 mM), Acetate buffer (20 mM), Mannitol (250 mM).

Composition according to the invention, characterized in that it comprises 3.00 mg/mL of pramlintide, 300 µg/mL of exenatide and m-cresol (20 mM), Acetate buffer (20 mM), Mannitol (250 mM).

Composition according to the invention, characterized in that it comprises 3.00 mg/mL of pramlintide, 360 µg/mL of exenatide and m-cresol (20 mM), Acetate buffer (20 mM), Mannitol (250 mM).

Composition according to the invention, characterized in that it comprises 4.00 mg/mL of pramlintide, 160 µg/mL of exenatide and m-cresol (20 mM), Acetate buffer (20 mM), Mannitol (250 mM).

Composition according to the invention, characterized in that it comprises 4.00 mg/mL of pramlintide, 240 µg/mL of exenatide and m-cresol (20 mM), Acetate buffer (20 mM), Mannitol (250 mM).

Composition according to the invention, characterized in that it comprises 4.00 mg/mL of pramlintide, 320 µg/mL of exenatide and m-cresol (20 mM), Acetate buffer (20 mM), Mannitol (250 mM).

Composition according to the invention, characterized in that it comprises 4.00 mg/mL of pramlintide, 400 µg/mL of exenatide and m-cresol (20 mM), Acetate buffer (20 mM), Mannitol (250 mM).

Composition according to the invention, characterized in that it comprises 4.00 mg/mL of pramlintide, 460 µg/mL of exenatide and m-cresol (20 mM), Acetate buffer (20 mM), Mannitol (250 mM).

Composition according to the invention, characterized in that it comprises 0.6 mg/mL of pramlintide and 60µg/mL of lixisenatide.

Composition according to the invention, characterized in that it comprises 0.6 mg/mL of pramlintide and 90 µg/mL of lixisenatide.

Composition according to the invention, characterized in that it comprises 0.6 mg/mL of pramlintide and 120 µg/mL of lixisenatide.

Composition according to the invention, characterized in that it comprises 0.6 mg/mL of pramlintide and 150 µg/mL of lixisenatide.

Composition according to the invention, characterized in that it comprises 0.6 mg/mL of pramlintide and 180 µg/mL of lixisenatide.

Composition according to the invention, characterized in that it comprises 1.00 mg/mL of pramlintide and 100 µg/mL of lixisenatide.

Composition according to the invention, characterized in that it comprises 1.00 mg/mL of pramlintide and 150 µg/mL of lixisenatide.

Composition according to the invention, characterized in that it comprises 1.00 mg/mL of pramlintide and 200 µg/mL of lixisenatide.

Composition according to the invention, characterized in that it comprises 1.00 mg/mL of pramlintide and 250 µg/mL of lixisenatide.

Composition according to the invention, characterized in that it comprises 1.00 mg/mL of pramlintide and 300 µg/mL of lixisenatide.

Composition according to the invention, characterized in that it comprises 2.00 mg/mL of pramlintide and 200 µg/mL of lixisenatide.

Composition according to the invention, characterized in that it comprises 2.00 mg/mL of pramlintide and 300 µg/mL of lixisenatide.

Composition according to the invention, characterized in that it comprises 2.00 mg/mL of pramlintide and 400 µg/mL of lixisenatide .

Composition according to the invention, characterized in that it comprises 2.00 mg/mL of pramlintide and 500 µg/mL of lixisenatide.

Composition according to the invention, characterized in that it comprises 2.00 mg/mL of pramlintide and 600 µg/mL of lixisenatide.

Composition according to the invention, characterized in that it comprises 3.00 mg/mL of pramlintide and 300 µg/mL of lixisenatide.

Composition according to the invention, characterized in that it comprises 3.00 mg/mL of pramlintide and 450 µg/mL of lixisenatide.

Composition according to the invention, characterized in that it comprises 3.00 mg/mL of pramlintide and 600 µg/mL of lixisenatide.

Composition according to the invention, characterized in that it comprises 3.00 mg/mL of pramlintide and 750 µg/mL of lixisenatide.

Composition according to the invention, characterized in that it comprises 3.00 mg/mL of pramlintide and 900 µg/mL of lixisenatide.

Composition according to the invention, characterized in that it comprises 4.00 mg/mL of pramlintide and 400 µg/mL of lixisenatide.

Composition according to the invention, characterized in that it comprises 4.00 mg/mL of pramlintide and 600 µg/mL of lixisenatide.

Composition according to the invention, characterized in that it comprises 4.00 mg/mL of pramlintide and 800 µg/mL of lixisenatide.

Composition according to the invention, characterized in that it comprises 4.00 mg/mL of pramlintide and 1000 µg/mL of lixisenatide.

Composition according to the invention, characterized in that it comprises 4.00 mg/mL of pramlintide and 1200 µg/mL of lixisenatide.

Composition according to the invention, characterized in that it comprises 0.6 mg/mL of pramlintide, 60µg/mL of lixisenatide and m-cresol (20 mM), Acetate buffer (20 mM), Mannitol (250 mM).

Composition according to the invention, characterized in that it comprises 0.6 mg/mL of pramlintide, 90 µg/mL of lixisenatide and m-cresol (20 mM), Acetate buffer (20 mM), Mannitol (250 mM).

Composition according to the invention, characterized in that it comprises 0.6 mg/mL of pramlintide, 120 µg/mL of lixisenatide and m-cresol (20 mM), Acetate buffer (20 mM), Mannitol (250 mM).

Composition according to the invention, characterized in that it comprises 0.6 mg/mL of pramlintide, 150 µg/mL of lixisenatide and m-cresol (20 mM), Acetate buffer (20 mM), Mannitol (250 mM).

Composition according to the invention, characterized in that it comprises 0.6 mg/mL of pramlintide, 180 µg/mL of lixisenatide and m-cresol (20 mM), Acetate buffer (20 mM), Mannitol (250 mM).

Composition according to the invention, characterized in that it comprises 1.00 mg/mL of pramlintide, 100 µg/mL of lixisenatide and m-cresol (20 mM), Acetate buffer (20 mM), Mannitol (250 mM).

Composition according to the invention, characterized in that it comprises 1.00 mg/mL of pramlintide, 150 µg/mL of lixisenatide and m-cresol (20 mM), Acetate buffer (20 mM), Mannitol (250 mM).

Composition according to the invention, characterized in that it comprises 1.00 mg/mL of pramlintide, 200 µg/mL of lixisenatide and m-cresol (20 mM), Acetate buffer (20 mM), Mannitol (250 mM).

Composition according to the invention, characterized in that it comprises 1.00 mg/mL of pramlintide, 250 µg/mL of lixisenatide and m-cresol (20 mM), Acetate buffer (20 mM), Mannitol (250 mM).

Composition according to the invention, characterized in that it comprises 1.00 mg/mL of pramlintide, 300 µg/mL of lixisenatide and m-cresol (20 mM), Acetate buffer (20 mM), Mannitol (250 mM).

Composition according to the invention, characterized in that it comprises 2.00 mg/mL of pramlintide, 200 µg/mL of lixisenatide and m-cresol (20 mM), Acetate buffer (20 mM), Mannitol (250 mM).

Composition according to the invention, characterized in that it comprises 2.00 mg/mL of pramlintide, 300 µg/mL of lixisenatide and m-cresol (20 mM), Acetate buffer (20 mM), Mannitol (250 mM).

Composition according to the invention, characterized in that it comprises 2.00 mg/mL of pramlintide, 400 µg/mL of lixisenatide and m-cresol (20 mM), Acetate buffer (20 mM), Mannitol (250 mM).

Composition according to the invention, characterized in that it comprises 2.00 mg/mL of pramlintide, 500 µg/mL of lixisenatide and m-cresol (20 mM), Acetate buffer (20 mM), Mannitol (250 mM).

Composition according to the invention, characterized in that it comprises 2.00 mg/mL of pramlintide, 600 µg/mL of lixisenatide and m-cresol (20 mM), Acetate buffer (20 mM), Mannitol (250 mM).

Composition according to the invention, characterized in that it comprises 3.00 mg/mL of pramlintide, 300 of lixisenatide and m-cresol (20 mM), Acetate buffer (20 mM), Mannitol (250 mM).

Composition according to the invention, characterized in that it comprises 3.00 mg/mL of pramlintide, 450 µg/mL of lixisenatide and m-cresol (20 mM), Acetate buffer (20 mM), Mannitol (250 mM).

Composition according to the invention, characterized in that it comprises 3.00 mg/mL of pramlintide, 600 µg/mL of lixisenatide and m-cresol (20 mM), Acetate buffer (20 mM), Mannitol (250 mM).

Composition according to the invention, characterized in that it comprises 3.00 mg/mL of pramlintide, 750 µg/mL of lixisenatide and m-cresol (20 mM), Acetate buffer (20 mM), Mannitol (250 mM).

Composition according to the invention, characterized in that it comprises 3.00 mg/mL of pramlintide, 900 µg/mL of lixisenatide and m-cresol (20 mM), Acetate buffer (20 mM), Mannitol (250 mM).

Composition according to the invention, characterized in that it comprises 4.00 mg/mL of pramlintide, 400 µg/mL of lixisenatide and m-cresol (20 mM), Acetate buffer (20 mM), Mannitol (250 mM).

Composition according to the invention, characterized in that it comprises 4.00 mg/mL of pramlintide, 600 µg/mL of lixisenatide and m-cresol (20 mM), Acetate buffer (20 mM), Mannitol (250 mM).

Composition according to the invention, characterized in that it comprises 4.00 mg/mL of pramlintide, 800 µg/mL of lixisenatide and m-cresol (20 mM), Acetate buffer (20 mM), Mannitol (250 mM).

Composition according to the invention, characterized in that it comprises 4.00 mg/mL of pramlintide, 1000 µg/mL of lixisenatide and m-cresol (20 mM), Acetate buffer (20 mM), Mannitol (250 mM).

Composition according to the invention, characterized in that it comprises 4.00 mg/mL of pramlintide, 1200 µg/mL of lixisenatide and m-cresol (20 mM), Acetate buffer (20 mM), Mannitol (250 mM).

The compositions according to the invention can moreover include excipients in compliance with the Pharmacopoeias, in particular the EP and/or US Pharmacopoeias.

According to an embodiment, the composition can be in solid or lyophilized form. This composition can then be used to reconstitute a solution or a formulation.

The methods of administration considered are the intravenous, subcutaneous, intradermal or intramuscular route.

According to an embodiment, the method of administration is the subcutaneous route.

The transdermal, oral, nasal, vaginal, ocular, buccal, pulmonary administration routes are also considered.

The invention also relates to single-dose formulations.

In an embodiment, the formulations are in the form of an injectable solution.

The preparation of a composition according to the invention has the advantage that it can be implemented by simple mixing of an aqueous solution of pramlintide and of exenatide or lixisenatide, in aqueous solution or in lyophilized form.

If necessary, the composition of the mixture is adjusted in terms of excipients such as glycerol, m-cresol and polysorbate 20 (Tween® 20). This addition can be carried out by addition of concentrated solutions of said excipients.

### Examples

### Compositions

### Example C1 : 250 µg/mL exenatide solution (Byetta®)

This solution is a commercial solution of exenatide marketed by the company NOVO NORDISK under the name of Byetta®. The excipients in this composition C1 are detailed in Table 1.

### Example C1' : Preparation of a 2 mg/mL exenatide solution at pH 4 (Composition C1')

Exenatide powder (obtained by peptide synthesis) is dissolved with a 10 mM HCl solution. An acetate buffer solution is then added to give the composition C1'. The composition is detailed in Table 1.

### Example C2: Preparation of a 250 µg/mL pramlintide solution at pH 4 (Composition C2).

A 5 mg/mL concentrated solution of pramlintide (obtained by peptide synthesis) and excipients is prepared via a stepwise addition in an empty container in the following order: m-cresol, acetate buffer, mannitol and pramlintide. Sufficient quantity of water is then added and the pH is adjusted to 4 to obtain composition C2 which is detailed in Table 1.

### Exemple C2' : Preparation of a 10 mg/mL pramlintide solution at pH 4 (Composition C2').

Pramlintide powder (obtained by peptide synthesis) is dissolved with a 10 mM HCl solution. An acetate buffer solution is then added to give the composition C2'. The composition is detailed in Table 1.

### Exemple C3 : Preparation of a 3 mg/mL lixisenatide solution at pH 4 (Composition C3).

Lixisenatide powder (obtained by peptide synthesis) is dissolved with a 10 mM HCl solution. An acetate buffer solution is then added to give the composition C3. The composition is detailed in Table 1.

### Example C4: Preparation of a stock solution of excipients (matrix) at pH 4 (Composition C4).

A concentrated solution of excipients is prepared via a stepwise addition in an empty container in the following order: m-cresol, acetate buffer and mannitol. Sufficient quantity of water is then added and the pH is adjusted to 4 to obtain Composition C4. This composition is detailed in Table 1.

### Example C5: Preparation of a dilution solution at pH 4 (Composition C5).

A concentrated solution of excipients is prepared via a stepwise addition in an empty container in the following order: BSA (Bovine Serum Albumine), acetate buffer, NaCl and polysorbate 20. Sufficient quantity of water is then added and the pH is adjusted to 4 to obtain Composition C5.

### Exemple C6 : Preparation of a solution of a vehicle (Composition C6).

To a volume VC4 of the composition C4, as prepared in example C4, is added a VC5 of the dilution solution C5, as prepared in example C5 to obtain the final solution C6 which composition is detailed in Table 1.

### Example C7 : Preparation of solutions of pramlintide alone or in combination with exenatide or lixisenatide at pH 4.

The dilution solution (Composition C5) is used to dilute the solution of pramlintide (Composition C2) and of exenatide (Composition C1 - Byetta) to prepare the solutions C7-1 to C7-8 as shown in Table 1 that will be injected to the animals during the in vivo assays.

The compositions of pramlintide and exenatide C7-9 to C7-33 are obtained by mixing a VC4 of composition C4, a volume VC1' or VC1 of the compostion of exenatide C1' or C1 and a VC2' of the composition of pramlintide C2'.

The compositions of pramlintide and lixisenatide C8-1 to C8-24 are obtained by mixing a VC4 of composition C4, a volume VC3 of the compostion of lixisenatide C3 and a VC2' of the composition of pramlintide C2'.

**Table 1: Stock solutions C1 to C4, dilute solution C5, vehicle solution C6, diluted solution of pramlintide C7-1 and C7-2 and compositions according to the invention C7-3 to C7-33 and C8-1 to C8-24 prepared to be injected to the animals in the in vivo assays.**

| **Composition** | **Pramlintide** (mg/mL) | **Exenatide** (µg/mL) | **Lixisenatide** (µg/mL) | **Excipients** |
|---|---|---|---|---|
| **C1** | - | 250 | - | m-cresol (20 mM) |
| | | | | Acetate buffer (20 mM) |
| | | | | Mannitol (250 mM) |
| **C1'** | - | 2000 | - | Acetate buffer (20 mM) |
| **C2** | 0.250 | - | - | m-cresol (20 mM) |
| | | | | Acetate buffer (20 mM) |
| | | | | Mannitol (250 mM) |
| **C2'** | 10 | - | - | Acetate buffer (20 mM) |
| **C3** | - | - | 3000 | Acetate buffer (20 mM) |
| **C4** | - | - | - | m-cresol (20 mM) |
| | | | | Acetate buffer (20 mM) |
| | | | | Mannitol (250 mM) |
| **C5** | - | - | - | Acetate buffer (20 mM) |
| | | | | BSA (1 mM) |
| | | | | NaCl (130 mM) |
| | | | | Polysorbate 20 (0.5 mM) |
| **C6** | - | - | - | m-cresol (3.3 mM) |
| | | | | Acetate buffer (20 mM) |
| | | | | Mannitol (42 mM) |
| | | | | BSA (0.83 mM) |
| | | | | NaCl (108 mM) |
| | | | | Polysorbate 20 (0.42 mM) |
| **C7-1** | 0.0042 | - | - | m-cresol (3.3 mM) |
| | | | | Acetate buffer (20 mM) |
| | | | | Mannitol (42 mM) |
| | | | | BSA (0.83 mM) |
| | | | | NaCl (108 mM) |
| | | | | Polysorbate 20 (0.42 mM) |
| **C7-2** | 0.042 | - | - | m-cresol (3.3 mM) |
| | | | | Acetate buffer (20 mM) |
| | | | | Mannitol (42 mM) |
| | | | | BSA (0.83 mM) |
| | | | | NaCl (108 mM) |
| | | | | Polysorbate 20 (0.42 mM) |
| **C7-3** | 0.0042 | 0.42 | - | m-cresol (3.4 mM) |
| | | | | Acetate buffer (20 mM) |
| | | | | Mannitol (42 mM) |
| | | | | BSA (0.83 mM) |
| | | | | NaCl (108 mM) |
| | | | | Polysorbate 20 (0.42 mM) |
| **C7-4** | 0.042 | 0.42 | - | m-cresol (3.4 mM) |
| | | | | Acetate buffer (20 mM) |
| | | | | Mannitol (42 mM) |
| | | | | BSA (0.83 mM) |
| | | | | NaCl (108 mM) |
| | | | | Polysorbate 20 (0.42 mM) |
| **C7-5** | 0.042 | 4.2 | - | m-cresol (3.4 mM) |
| | | | | Acetate buffer (20 mM) |
| | | | | Mannitol (42 mM) |
| | | | | BSA (0.83 mM) |
| | | | | NaCl (108 mM) |
| | | | | Polysorbate 20 (0.42 mM) |
| **C7-6** | 0.042 | 0.83 | - | m-cresol (3.4 mM) |
| | | | | Acetate buffer (20 mM) |
| | | | | Mannitol (43 mM) |
| | | | | BSA (0.83 mM) |
| | | | | NaCl (108 mM) |
| | | | | Polysorbate 20 (0.42 mM) |
| **C7-7** | 0.042 | 3.3 | - | m-cresol (3.6 mM) |
| | | | | Acetate buffer (20 mM) |
| | | | | Mannitol (45 mM) |
| | | | | BSA (0.82 mM) |
| | | | | NaCl (107 mM) |
| | | | | Polysorbate 20 (0.41 mM) |
| **C7-8** | 0.042 | 8.3 | - | m-cresol (4.0 mM) |
| | | | | Acetate buffer (17 mM) |
| | | | | Mannitol (50 mM) |
| | | | | BSA (0.67 mM) |
| | | | | NaCl (87 mM) |
| | | | | Polysorbate 20 (0.33 mM) |
| **C7-9** | 0.6 | 24 | - | m-cresol (20 mM) |
| | | | | Acetate buffer (20 mM) |
| | | | | Mannitol (250 mM) |
| **C7-10** | 0.6 | 36 | - | m-cresol (20 mM) |
| | | | | Acetate buffer (20 mM) |
| | | | | Mannitol (250 mM) |
| **C7-11** | 0.6 | 48 | - | m-cresol (20 mM) |
| | | | | Acetate buffer (20 mM) |
| | | | | Mannitol (250 mM) |
| **C7-12** | 0.6 | 60 | - | m-cresol (20 mM) |
| | | | | Acetate buffer (20 mM) |
| | | | | Mannitol (250 mM) |
| **C7-13** | 0.6 | 72 | - | m-cresol (20 mM) |
| | | | | Acetate buffer (20 mM) |
| | | | | Mannitol (250 mM) |
| **C7-14** | 1 | 40 | - | m-cresol (20 mM) |
| | | | | Acetate buffer (20 mM) |
| | | | | Mannitol (250 mM) |
| **C7-15** | 1 | 60 | - | m-cresol (20 mM) |
| | | | | Acetate buffer (20 mM) |
| | | | | Mannitol (250 mM) |
| **C7-16** | 1 | 80 | - | m-cresol (20 mM) |
| | | | | Acetate buffer (20 mM) |
| | | | | Mannitol (250 mM) |
| **C7-17** | 1 | 100 | - | m-cresol (20 mM) |
| | | | | Acetate buffer (20 mM) |
| | | | | Mannitol (250 mM) |
| **C7-18** | 1 | 120 | - | m-cresol (20 mM) |
| | | | | Acetate buffer (20 mM) |
| | | | | Mannitol (250 mM) |
| **C7-19** | 2 | 80 | - | m-cresol (20 mM) |
| | | | | Acetate buffer (20 mM) |
| | | | | Mannitol (250 mM) |
| **C7-20** | 2 | 120 | - | m-cresol (20 mM) |
| | | | | Acetate buffer (20 mM) |
| | | | | Mannitol (250 mM) |
| **C7-21** | 2 | 160 | - | m-cresol (20 mM) |
| | | | | Acetate buffer (20 mM) |
| | | | | Mannitol (250 mM) |
| **C7-22** | 2 | 200 | - | m-cresol (20 mM) |
| | | | | Acetate buffer (20 mM) |
| | | | | Mannitol (250 mM) |
| **C7-23** | 2 | 240 | - | m-cresol (20 mM) |
| | | | | Acetate buffer (20 mM) |
| | | | | Mannitol (250 mM) |
| **C7-24** | 3 | 120 | - | m-cresol (20 mM) |
| | | | | Acetate buffer (20 mM) |
| | | | | Mannitol (250 mM) |
| **C7-25** | 3 | 180 | - | m-cresol (20 mM) |
| | | | | Acetate buffer (20 mM) |
| | | | | Mannitol (250 mM) |
| **C7-26** | 3 | 240 | - | m-cresol (20 mM) |
| | | | | Acetate buffer (20 mM) |
| | | | | Mannitol (250 mM) |
| **C7-27** | 3 | 300 | - | m-cresol (20 mM) |
| | | | | Acetate buffer (20 mM) |
| | | | | Mannitol (250 mM) |
| **C7-28** | 3 | 360 | - | m-cresol (20 mM) |
| | | | | Acetate buffer (20 mM) |
| | | | | Mannitol (250 mM) |
| **C7-29** | 4 | 160 | - | m-cresol (20 mM) |
| | | | | Acetate buffer (20 mM) |
| | | | | Mannitol (250 mM) |
| **C7-30** | 4 | 240 | - | m-cresol (20 mM) |
| | | | | Acetate buffer (20 mM) |
| | | | | Mannitol (250 mM) |
| **C7-31** | 4 | 320 | - | m-cresol (20 mM) |
| | | | | Acetate buffer (20 mM) |
| | | | | Mannitol (250 mM) |
| **C7-32** | 4 | 400 | - | m-cresol (20 mM) |
| | | | | Acetate buffer (20 mM) |
| | | | | Mannitol (250 mM) |
| **C7-33** | 4 | 460 | - | m-cresol (20 mM) |
| | | | | Acetate buffer (20 mM) |
| | | | | Mannitol (250 mM) |
| **C8-1** | 0.6 | - | 60 | m-cresol (20 mM) |
| | | | | Acetate buffer (20 mM) |
| | | | | Mannitol (250 mM) |
| **C8-1** | 0.6 | - | 90 | m-cresol (20 mM) |
| | | | | Acetate buffer (20 mM) |
| | | | | Mannitol (250 mM) |
| **C8-2** | 0.6 | - | 120 | m-cresol (20 mM) |
| | | | | Acetate buffer (20 mM) |
| | | | | Mannitol (250 mM) |
| **C8-3** | 0.6 | - | 150 | m-cresol (20 mM) |
| | | | | Acetate buffer (20 mM) |
| | | | | Mannitol (250 mM) |
| **C8-4** | 0.6 | - | 180 | m-cresol (20 mM) |
| | | | | Acetate buffer (20 mM) |
| | | | | Mannitol (250 mM) |
| **C8-5** | 1 | - | 100 | m-cresol (20 mM) |
| | | | | Acetate buffer (20 mM) |
| | | | | Mannitol (250 mM) |
| **C8-6** | 1 | - | 150 | m-cresol (20 mM) |
| | | | | Acetate buffer (20 mM) |
| | | | | Mannitol (250 mM) |
| **C8-7** | 1 | - | 200 | m-cresol (20 mM) |
| | | | | Acetate buffer (20 mM) |
| | | | | Mannitol (250 mM) |
| **C8-8** | 1 | - | 250 | m-cresol (20 mM) |
| | | | | Acetate buffer (20 mM) |
| | | | | Mannitol (250 mM) |
| **C8-9** | 1 | - | 300 | m-cresol (20 mM) |
| | | | | Acetate buffer (20 mM) |
| | | | | Mannitol (250 mM) |
| **C8-10** | 2 | - | 200 | m-cresol (20 mM) |
| | | | | Acetate buffer (20 mM) |
| | | | | Mannitol (250 mM) |
| **C8-11** | 2 | - | 300 | m-cresol (20 mM) |
| | | | | Acetate buffer (20 mM) |
| | | | | Mannitol (250 mM) |
| **C8-12** | 2 | - | 400 | m-cresol (20 mM) |
| | | | | Acetate buffer (20 mM) |
| | | | | Mannitol (250 mM) |
| **C8-13** | 2 | - | 500 | m-cresol (20 mM) |
| | | | | Acetate buffer (20 mM) |
| | | | | Mannitol (250 mM) |
| **C8-14** | 2 | - | 600 | m-cresol (20 mM) |
| | | | | Acetate buffer (20 mM) |
| | | | | Mannitol (250 mM) |
| **C8-15** | 3 | - | 300 | m-cresol (20 mM) |
| | | | | Acetate buffer (20 mM) |
| | | | | Mannitol (250 mM) |
| **C8-16** | 3 | - | 450 | m-cresol (20 mM) |
| | | | | Acetate buffer (20 mM) |
| | | | | Mannitol (250 mM) |
| **C8-17** | 3 | - | 600 | m-cresol (20 mM) |
| | | | | Acetate buffer (20 mM) |
| | | | | Mannitol (250 mM) |
| **C8-18** | 3 | - | 750 | m-cresol (20 mM) |
| | | | | Acetate buffer (20 mM) |
| | | | | Mannitol (250 mM) |
| **C8-19** | 3 | - | 900 | m-cresol (20 mM) |
| | | | | Acetate buffer (20 mM) |
| | | | | Mannitol (250 mM) |
| **C8-20** | 4 | - | 400 | m-cresol (20 mM) |
| | | | | Acetate buffer (20 mM) |
| | | | | Mannitol (250 mM) |
| **C8-21** | 4 | - | 600 | m-cresol (20 mM) |
| | | | | Acetate buffer (20 mM) |
| | | | | Mannitol (250 mM) |
| **C8-22** | 4 | - | 800 | m-cresol (20 mM) |
| | | | | Acetate buffer (20 mM) |
| | | | | Mannitol (250 mM) |
| **C8-23** | 4 | - | 1000 | m-cresol (20 mM) |
| | | | | Acetate buffer (20 mM) |
| | | | | Mannitol (250 mM) |
| **C8-24** | 4 | - | 1200 | m-cresol (20 mM) |
| | | | | Acetate buffer (20 mM) |
| | | | | Mannitol (250 mM) |

### In vivo studies

### Effects of pramlintide and pramlintide with exenatide compositions on gastric emptying

The aim of this study was to evaluate the effects of pramlintide compositions C7-1 and C7-2 and pramlintide combined with exenatide compositions (C7-3 to C7-5) on gastro-intestinal transit (gastric emptying) in the mouse.

### Example D1 : General procedure for in vivo studies of compositions of pramlintide alone and pramlintide in combination with exenatide

Studies were conducted on naïve male Swiss mice (Janvier Lab) as no gender specific effect were expected. Body weight range of 25-32 g were included in the analysis.

Mice were approximately fasted for 18 hours (∼T0-18 h). The gastric emptying (i.e. stomach content) was measured via the administration of a suspension of charcoal at TO (activated charcoal (0.4 ml/mouse, ∼0.4mg/mouse; Sigma Reference number C9157), suspended 10% with 2.5% arabic gum in distilled water). The studied composition or a vehicle (as a standard control) was administered subcutaneously using Hamilton syringes 20 minutes before charcoal (T0-20 min), then 50 minutes later (T0+50 min), they were sacrificed, and the stomach was removed. The stomach was excised from the abdomen and weighed with its contents (full stomach weight). Afterwards, the stomach was opened, the contents washed out and the weight recorded again (empty stomach weight).

The results are expressed as the variation of gastric content with the administration compositions comprising pramlintide compared with the gastric content with administration the vehicle (weight of gastric content with pramlintide / weight of gastric content with vehicle).

### Example D2 : Gastric emptying measurements with compositions comprising pramlintide alone

Measurements of the gastric emptying for compositions C7-2 were obtained according to the general procedure. Five different doses of pramlintide were studied. Each dose was tested on 4 mice. The results of this study are presented in table 2.

**Table 2: Effect of compositions of pramlintide alone on the gastric content**

| Composition | Dose of pramlintide (µg/kg) | Concentration of pramlintide (µg/mL) | Variability of the stomach content weight compared to the vehicle |
|---|---|---|---|
| C6 (Vehicle) | -* | 0 | - |
| C7-2 | 50 | 42 | +209% (± 10%) |
| C7-2 | 80 | 42 | +166% (± 25%) |
| C7-2 | 150 | 42 | +233% (± 20%) |
| C7-2 | 250 | 42 | +144% (± 20%) |
| C7-2 | 350 | 42 | +205% (± 30%) |

| | | | |
|---|---|---|---|
| * Vehicle administered | | | |

These examples demonstrate that subcutaneous injection of a composition comprising pramlintide alone increases the stomach content weight i.e. slows down the gastric emptying.

### 1.

### Example D3 : Gastric emptying measurements of compositions Comprising a combination of pramlintide and exenatide at different doses compared to compositions of pramlintide alone

Measurements of the gastric emptying for compositions C6 and C7-1 to C7-5 were obtained according to the general procedure described in exemple D1. Two sets of experiments were carried out. Each dose was tested on 8 mice apart for composition C7-2 that was tested on 10 mice. The results are presented in table 3.

**Table 3 : Effect of compositions of pramlintide/exenatide on the gastric content**

| Composition | Pramlintide (µg/kg) | Exenatide (µg/kg) | Variability of the stomach content weight compared to the vehicle | Variability of the stomach content weight compared to the content weight of the composition of pramlintide alone at the same dose |
|---|---|---|---|---|
| C6 | -* | | - | - |
| C7-1 | 12.5 | - | +158% (± 10%) | - |
| C7-3 | 12.5 | 0.125 | +100% (± 10%) | -22% |
| C7-2 | 25 | - | +118% (± 15%) | - |
| C7-4 | 25 | 0.25 | +7.5% (± 15%) | -49% |
| C7-4 | 50 | 0.5 | +107% (±20%) | - |
| C6 | -* | - | - | - |
| C7-2 | 25 | - | +75% (± 15%) | |
| C7-5 | 25 | 5 | -18% (± 10%) | -53% |
| C7-5 | 25 | 2 | -24% (± 20%) | -57% |
| C7-4 | 25 | 0.5 | +8% | -38% |

| | | | | |
|---|---|---|---|---|
| * Vehicle administered | | | | |

These results show that pramlintide alone increases the gastric content weight i.e. slows down the gastric emptying and that the addition of exenatide to pramlintide decreases the gastric content weight in comparison with the same composition without exenatide i.e. increase the gastric emptying.

## Claims

1. Composition comprising pharmaceutical active ingredients (API) chosen in the group comprising pramlintide and a GLP-1 receptor agonist (GLP-1 RA).

2. Composition according to claim 1, **characterized in that** the GLP-1 receptor agonist (GLP-1 RA) is chosen amongst lixisenatide and exenatide.

3. Composition according to any one of the preceding claims, **characterized in that** the GLP-1 receptor agonist (GLP-1 RA) is exenatide.

4. Composition according to any one of the preceding claims, **characterized in that** it is in the form of an injectable aqueous formulation.

5. Composition according to claim 4, **characterized in that** its pH is ranging from 3.5 to 4.4.

6. Composition according to any one of the preceding claims, **characterized in that** it comprises a concentration of pramlintide ranging from 0.5 to 10 mg/ml.

7. Composition according to any one of the preceding claims, **characterized in that** it comprises a concentration of exenatide ranging from 20 to 500 µg/ml.

8. Composition according to any one of the preceding claims, **characterized in that** ratio pramlintide / exenatide (weight/weight) is ranging from 2 to 50.

9. Composition according to any one of the preceding claims, for use in the method for the treatment of obesity, overweight and/or diabetes, in particular type 2 diabetes, **characterized in that** it reduces the slowing effect of gastric emptying of amylin RA as compared to the same composition without the corresponding GLP-1 RA.

10. Composition according to to any one of the preceding claims, for the treatment of obesity and/or diabetes, in particular Type 2 Diabetes, is **characterized in that** the the daily dose of pramlintide is ranging from 150 to 2000 µg/day

11. Compositions according to any of the preceding claims, **characterized in that** the daily dose of exenatide is ranging from 10 to 250 µg.
